# EUROPEAN PATENT SPECIFICATION

(11) **EP 3 406 735 B1**
(45) Date of publication and mention of the grant of the patent: **25.11.2020**
(21) Application number: 17741546.0
(22) Date of filing: 20.01.2017
(51) Int. Cl.: C12Q 1/68, C12M 1/00, C12N 15/09, G01N 33/53, G01N 33/543, C12N 9/02

(54) **TARGET ANALYSIS METHOD AND TARGET ANALYSIS KIT FOR USE IN SAID METHOD**
ZIELANALYSEVERFAHREN UND ZIELANALYSEKIT ZUR VERWENDUNG IN DIESEM VERFAHREN
PROCÉDÉ D'ANALYSE CIBLE ET TROUSSE D'ANALYSE CIBLE DESTINÉE À L'UTILISATION DANS LEDIT PROCÉDÉ

(30) Priority: 22.01.2016 JP 2016011024
(43) Date of publication of application: 28.11.2018
(73) Proprietor: NEC Solution Innovators, Ltd., Koto-ku, Tokyo 136-8627 (JP)
(72) Inventor: YOSHIDA, Yoshihito, Tokyo 136-8627 (JP); HORII, Katsunori, Tokyo 136-8627 (JP); AKITOMI, Jou, Tokyo 136-8627 (JP); KANEKO, Naoto, Tokyo 136-8627 (JP); SHIMIZU, Akihisa, Tokyo 136-8627 (JP); FUJITA, Tomoko, Tokyo 136-8627 (JP); WAGA, Iwao, Tokyo 136-8627 (JP)
(74) Representative: Betten & Resch
(86) International application number: PCT/JP2017/001958
(87) International publication number: WO 2017/126669

(56) References cited:
- EP-A1- 3 249 404
- WO-A1-2007/086403
- WO-A1-2007/086403
- WO-A1-2015/083391
- WO-A1-2015/083391
- WO-A1-2016/117701
- JP-A- 2009 201 406
- JP-A- 2009 201 406
- SONIA AMAYA-GONZ?LEZ ET AL: "Aptamer-Based Analysis: A Promising Alternative for Food Safety Control", SENSORS, vol. 13, no. 12, 28 November 2013 (2013-11-28), pages 16292-16311, XP055233452, DOI: 10.3390/s131216292

## Description

### TECHNICAL FIELD

The present invention relates to a target analysis method and the use of a target analysis kit in the method.

### BACKGROUND ART

Target analyses are performed in various fields such as clinical medical care, food, and environment. As a method for the target analysis, for example, a method using immunochromatography is known. However, because the immunochromatography requires the analysis time of about 15 to 20 minutes, there is a demand for a quicker analysis. Amaya-González et al., 2007, SENSORS, vol. 13, no. 12, discloses and reviews methods for detecting targets in samples using inter alia displacement methods encompassing aptamers, beads and labels.

### SUMMARY OF INVENTION

### Technical Problem

Hence, the present invention is intended to provide a new target analysis method that can analyze a target quickly and the use of a target analysis kit in the method.

### Solution to Problem

The present invention provides a target analysis method (hereinafter, also referred to as an "analysis method") including: causing a specimen, a labeled binding nucleic acid molecule that binds to a target (hereinafter, also referred to as a "binding nucleic acid molecule"), and a carrier on which a blocking nucleic acid molecule that binds to the labeled binding nucleic acid molecule is immobilized to react; separating a fraction of the carrier and a fraction of components other than the carrier from each other; and detecting a label of the labeled binding nucleic acid molecule in at least one of the fraction of the carrier or the fraction of components other than the carrier to analyze a target in the specimen, wherein the labeled binding nucleic acid molecule includes the following polynucleotide (a):
(a) the following polynucleotide (a1) or (a2):
   (a1) a polynucleotide consisting of a base sequence of SEQ ID NO: 1, and
   (a2) a polynucleotide that binds to a peanut allergen and is consisting of a base sequence having at least 90% identity to the base sequence of the polynucleotide (a1).

The present invention also provides a target analysis kit (hereinafter, also referred to as an "analysis kit") including: a labeled binding nucleic acid molecule that binds to a target; and a carrier on which a blocking nucleic acid molecule that binds to the labeled binding nucleic acid molecule is immobilized, wherein the kit is for use in the analysis method of the present invention.

### Advantageous Effects of Invention

According to the target analysis method and the use of an analysis kit in the method of the present invention, a target can be analyzed quickly.

### BRIEF DESCRIPTION OF DRAWINGS

[FIGs. 1A and 1B] FIGs. 1A and 1B are graphs each showing the amount of light emission in Example 1 of the present invention.
[FIGs. 2A to 2C] FIGs. 2A to 2C are graphs each showing the amount of light emission in Example 2 of the present invention.
[FIGs. 3A and 3B] FIGs. 3A and 3B are graphs each showing the amount of light emission in Example 3 of the present invention.
[FIG. 4] FIG. 4 is a graph showing the amount of light emission in Example 4 of the present invention.
[FIG. 5] FIG. 5 is a graph showing the amount of light emission in Example 5 of the present invention.
[FIG. 6] FIG. 6 is a graph showing the amount of light emission in Example 6 of the present invention.
[FIG. 7] FIG. 7 is a graph showing the amount of light emission in Example 7 of the present invention.

### DESCRIPTION OF EMBODIMENTS

The analysis method of the present invention includes: causing the specimen to react with the labeled binding nucleic acid molecule; and causing a mixture of the specimen and the labeled binding nucleic acid molecule to react with the carrier, for example.

In the analysis method and the use of an analysis kit of the present invention, for example, the blocking nucleic acid molecule is a nucleic acid molecule that includes a base sequence complementary to the labeled binding nucleic acid molecule.

In the analysis method and the use of an analysis kit of the present invention, for example, the blocking nucleic acid molecule is a nucleic acid molecule that includes a base sequence complementary to a base sequence that binds to the target in the labeled binding nucleic acid molecule.

In the analysis method and the use of an analysis kit of the present invention, for example, the base length of the blocking nucleic acid molecule is in the range from 1/1 to 1/20 of the base length of the labeled binding nucleic acid molecule.

In the analysis method and the use of an analysis kit of the present invention, for example, the blocking nucleic acid molecule is a nucleic acid molecule that includes a polynucleotide consisting of a base sequence having complementarity in a range from 5% to 100% with respect to a base sequence of the labeled binding nucleic acid molecule.

In the analysis method and the use of an analysis kit of the present invention, the labeled binding nucleic acid molecule includes the following polynucleotide (a):
(a) the following polynucleotide (a1):
   (a1) a polynucleotide consisting of a base sequence of SEQ ID NO: 1.

In the analysis method and the use of an analysis kit of the present invention, the blocking nucleic acid molecule includes the following polynucleotide (b):
(b) the following polynucleotide (b1):
(b1) a polynucleotide consisting of a base sequence of SEQ ID NO: 3.

In the analysis method and the use of an analysis kit of the present invention, for example, the carrier is a bead and is preferably a polystyrene bead. The bead is, for example, a magnetic bead.

In the analysis method of the present invention, for example, the fraction of the magnetic bead and the fraction of components other than the magnetic bead are separated from each other by separating the magnetic bead using a magnetic body.

In the analysis method of the present invention, for example, the label is an enzyme and an enzymatic reaction of the labeled binding nucleic acid molecule in at least one of the fraction of the carrier and the fraction of components other than the carrier is detected, and preferably, the enzymatic reaction is detected in the presence of a substrate for the enzyme.

In the analysis method of the present invention, for example, the enzyme is luciferase.

In the analysis method of the present invention, for example, the specimen is a food-derived specimen.

In the analysis method and the use of an analysis kit of the present invention, for example, the target is a peanut allergen. The peanut allergen is, for example, conarachin or a subunit thereof. The subunit is, for example, Ara h1.

In the analysis method and the use of an analysis kit of the present invention, for example, the peanut allergen is a native allergen or a thermally denatured allergen.

In the use of an analysis kit of the present invention, for example, the label is an enzyme, and is preferably luciferase.

The use of an analysis kit of the present invention further includes a substrate for the enzyme, for example.

### <Target analysis method>

The target analysis method of the present invention is, as described above,
characterized in that it includes: causing a specimen, a labeled binding nucleic acid molecule that binds to a target, and a carrier on which a blocking nucleic acid molecule that binds to the labeled binding nucleic acid molecule is immobilized to react (reaction step); separating a fraction of the carrier and a fraction of components other than the carrier from each other (separation step); and detecting a label of the labeled binding nucleic acid molecule in at least one of the fraction of the carrier and the fraction of components other than the carrier to analyze a target in the specimen (analysis step), wherein the labeled binding nucleic acid molecule includes the following polynucleotide (a):
(a) the following polynucleotide (a1) or (a2):
   (a1) a polynucleotide consisting of a base sequence of SEQ ID NO: 1, and
   (a2) a polynucleotide that binds to a peanut allergen and is consisting of a base sequence having at least 90% identity to the base sequence of the polynucleotide (a1).

The analysis method of the present invention is characterized in that it uses the labeled binding nucleic acid molecule and a carrier on which the blocking nucleic acid molecule is immobilized and performs the reaction step, the separation step, and the analysis step. Other steps and conditions are by no means limited. According to the analysis method of the present invention, for example, a target can be analyzed with high analysis sensitivity in a very short time of about 3 minutes. Furthermore, according to the analysis method of the present invention, for example, although the mechanism is unknown, a target can be analyzed with high analysis accuracy (specificity), for example, because nonspecific binding of the binding nucleic acid molecule can be suppressed.

The analysis method of the present invention may be, for example, a qualitative analysis to analyze the presence or absence of a target in the specimen or a quantitative analysis to analyze a degree (e.g. amount) of a target in the specimen.

In the analysis method of the present invention, the specimen to be subjected to the analysis is not particularly limited, and may be a food-derived specimen, for example. Examples of the food-derived specimen include foods, food ingredients, food additives, attached substances in food-processing factories, kitchens, etc., and liquid obtained after washing food-processing factories, kitchens, etc. The form of the specimen is not particularly limited, and the specimen may be liquid or solid, for example. When the specimen is solid, the specimen may be in the form of a mixed solution, an extract, a dissolved solution, or the like prepared using a solvent, for example. The solvent is not particularly limited, and may be, for example, water, physiological saline, or a buffer. The specimen may or may not contain the target, or whether the specimen contains the target may be unknown, for example.

The target is not particularly limited and may be any target. When the specimen is a food-derived specimen, the target may be, for example, food allergens. Specific examples of the food allergen include peanut allergens, but wheat allergens, milk allergens, egg allergens, soba allergens, shrimp allergens, and soybean allergens are also possible. Examples of the peanut allergen include conarachin, which is a main allergen of the peanut, and subunits and domains of the conarachin. Examples of the conarachin include conarachin I and conarachin II (a-conarachin). Examples of the subunit of the conarachin include Ara h1, Ara h2, and Ara h6. Examples of the wheat allergen include glutenin, gluten, gliadin, ω-5 gliadin, and subunits and domains of these allergens. Examples of the milk allergen include casein, α casein, s1 casein, β lactoglobulin, and subunits and domains of these allergens. Examples of the egg allergen include ovomucoid and ovotransferrin, which are main allergens of the egg, and subunits and domains of these allergens. Examples of the soba allergen include Fage 2 and subunits and domains of the Fage 2. Examples of the shrimp allergen include tropomyosin, which is a main allergen of the shrimp, and subunits and domains of the tropomyosin. Specific examples of the tropomyosin include Pen a 1, Pen i 1, and Met e 1. Examples of the soybean allergen include β-conglycinin, which is a main allergen of the soybean, and subunits and domains of the β-conglycinin. The allergens may be, for example, native allergens or thermally denatured allergens.

The labeled binding nucleic acid molecule is a nucleic acid molecule that binds to a target and is labeled. The binding nucleic acid molecule is as described above, and examples thereof include aptamers that bind to the targets. The binding between the binding nucleic acid molecule and the target can be examined, for example, by Surface Plasmon resonance (SPR) molecular interaction analysis or the like. For the analysis, for example, ProteON (product name, BioRad) can be used.

In the case where the target is a peanut allergen, the binding nucleic acid molecule is preferably a nucleic acid molecule that binds significantly specifically to the peanut allergen as compared to a soybean protein, for example.

According to the present invention, a binding nucleic acid molecule that binds to the peanut allergen may be, for example, a nucleic acid molecule including the following polynucleotide (a).
(a) at least one polynucleotide selected from the group consisting of the following polynucleotides (a1) and (a2):
   (a1) a polynucleotide consisting of the base sequence of SEQ ID NO: 1, and
   (a2) a polynucleotide that binds to a peanut allergen and is consisting of a base sequence having at least 90% identity to the base sequence of the polynucleotide (a1).

The polynucleotide (a1) is a polynucleotide consisting of the base sequence of SEQ ID NO: 1.
Peanut-binding nucleic acid molecule 1 (SEQ ID NO: 1)

In the polynucleotide (a2), the "identity" may be in any range as long as it allows the polynucleotide (a2) to bind to a peanut allergen, for example. The identity is, for example, at least 90%, at least 95%, at least 96%, at least 97%, at least 98%, or at least 99%. The "identity" may be calculated with analysis software such as BLAST or FASTA using default parameters, for example (hereinafter, the same applies).

As used herein, " a polynucleotide that hybridizes to a polynucleotide consisting of the base sequence of the polynucleotide (a1)" is, for example, a polynucleotide completely or partially complementary to the polynucleotide (a1). The hybridization can be detected, for example, by various hybridization assays. The hybridization assay is not particularly limited, and may be, for example, the method described in Sambrook et al. "Molecular Cloning: A Laboratory Manual 2nd Ed." [Cold Spring Harbor Laboratory Press (1989)].

As disclosed herein, a" stringent condition" may be any of a low stringent condition, a middle stringent condition, and a high stringent condition, for example. The "low stringent condition" refers to, for example, a condition in which 5 × SSC, 5 × Denhardt's solution, 0.5% SDS, and 50% formamide are used at 32°C. The "middle stringent condition" refers to, for example, a condition in which 5 × SSC, 5 × Denhardt's solution, 0.5% SDS, and 50% formamide are used at 42°C. The "high stringent condition" refers to, for example, a condition in which 5 × SSC, 5 × Denhardt's solution, 0.5% SDS, and 50% formamide are used at 50°C. Those skilled in the art can adjust the degree of the stringency, for example, by appropriately selecting the conditions such as a temperature, a salt concentration, the concentration and the length of a probe, an ionic strength, time, and the like. The "stringent condition" may be, for example, the condition described in Sambrook et al. "Molecular Cloning: A Laboratory Manual 2nd Ed." [Cold Spring Harbor Laboratory Press (1989)].

As disclosed herein, "a sequence is complementary to another sequence" means, for example, that annealing can be caused between these sequences. The term "complementary" means, for example, that complementarity between aligned two kinds of sequences is, for example, at least 90%, at least 95%, at least 96%, at least 97%, at least 98%, or at least 99%, and is preferably 100% (i.e., complete complementation). Furthermore, "a sequence is complementary to another sequence" means, for example, that the bases of a sequence from the 5' side to the 3' side and the bases of another sequence from the 3' side to the 5' side are complementary to each other.

The binding nucleic acid molecule may include one base sequence of any one of the polynucleotides (a1) to (a2) or two or more base sequences of the polynucleotides (a1) to (a2), for example. In the latter case, preferably, two or more polynucleotides are linked to form a single-stranded polynucleotide. The two or more polynucleotides may be directly linked or indirectly linked through linkers, for example. The linker is described in detail below. The polynucleotides are preferably linked directly or indirectly at their ends. The two or more polynucleotides may be identical to one another or different from one another, for example. The two or more polynucleotides are preferably identical to one another, for example. In the case where there are two or more polynucleotides, the number of polynucleotides is not particularly limited, and is, for example, 2 or more, and specifically, for example, 2 to 20, 2 to 10, 2, or 3.

The labeled binding nucleic acid molecule is, for example, a binding nucleic acid molecule labeled with a labeling substance. The labeling substance is not particularly limited, and examples thereof include enzymes, fluorescent substances, dyes, and isotopes. The enzyme is not particularly limited, and examples thereof include luciferase, alkaline phosphatase, peroxidase, β-galactosidase, and glucuronidase. The enzyme is preferably luciferase because it improves analysis sensitivity. Examples of the fluorescent substance include pyrene; TAMRA; fluorescein; Cy®3 dyes; Cy®5 dyes; FAM dyes; rhodamine dyes; Texas red dyes; and fluorophores such as JOE, MAX, HEX, and TYE. Examples of the dye include Alexa dyes such as Alexa® 488 and Alexa® 647.

The labeling substance is preferably bound to at least one of the 5' end and the 3' end of the binding nucleic acid molecule and is more preferably bound to the 5' end, for example. The labeling substance may be directly linked to the binding nucleic acid molecule or indirectly linked to the binding nucleic acid molecule through a linker for example.

The linker is not particularly limited and may be, for example, a non-nucleic acid molecule or a nucleic acid molecule. In the former case, the non-nucleic acid molecule may be, for example, the combination of avidin and biotin, the combination of a molecule including an amino group and a molecule including a carboxyl group, and the like. In the latter case, the structural unit of the linker is, for example, a nucleotide residue. Examples of the nucleotide residue include deoxyribonucleotide residues and ribonucleotide residues. The linker is not particularly limited, and examples thereof include polynucleotides such as DNA consisting of deoxyribonucleotide residues and DNA containing ribonucleotide residues. Specific examples of the linker include poly-deoxythymine (poly dT), poly-deoxyadenine (poly dA), and poly dAdT, which is a repetitive sequence of A and T. The linker is preferably poly dT or poly dAdT. The length of the linker is not particularly limited, and is, for example, 1 to 200-mer, 1 to 20-mer, 3 to 12-mer, or 5 to 9-mer.

The blocking nucleic acid molecule is a nucleic acid molecule that binds to the binding nucleic acid molecule. The blocking nucleic acid molecule is, for example, a nucleic acid molecule that inhibits binding between the target and the binding nucleic acid molecule. The blocking nucleic acid molecule may be, for example, a nucleic acid molecule that specifically binds to the binding nucleic acid molecule. A specific example of the blocking nucleic acid molecule is a nucleic acid molecule including a base sequence complementary to the binding nucleic acid molecule. The blocking nucleic acid molecule is, for example, completely or partially complementary to the binding nucleic acid molecule. Specifically, in the latter case, the blocking nucleic acid molecule may be, for example, a nucleic acid molecule including a polynucleotide consisting of a base sequence having complementarity in the range from 5 to 100%, 5 to 50%, or 5 to 20% with respect to the base sequence of the binding nucleic acid molecule.

The blocking nucleic acid molecule may be, for example, a base sequence complementary to the base sequence on the 3' end side of the binding nucleic acid molecule, the base sequence on the 5' end side of the binding nucleic acid molecule, or the base sequence of a region of the binding nucleic acid molecule other than these. The base sequence on the 3' end side is, for example, a base sequence continuous or discontinuous from the base on the 3' end of the binding nucleic acid molecule. Furthermore, the base sequence on the 5' end side is, for example, a base sequence continuous or discontinuous from the base on the 5' end of the binding nucleic acid molecule. The method for immobilizing the blocking nucleic acid molecule is not particularly limited and a publicly known method for immobilizing a nucleic acid molecule can be used.

The blocking nucleic acid molecule is preferably a nucleic acid molecule including a base sequence complementary to a base sequence (binding sequence) that binds to the target in the labeled binding nucleic acid molecule, for example. The blocking nucleic acid molecule may be completely or partially complementary to the binding sequence, for example. In the labeled binding nucleic acid molecule, the binding sequence is not particularly limited and can be determined appropriately depending on the base sequence of the binding nucleic acid molecule. In the case where the binding nucleic acid molecule is a polynucleotide consisting of the base sequence of SEQ ID NO: 1, the binding sequence may be, for example, the underlined base sequence in the base sequence of the SEQ ID NO: 1.

The ratio between the base length of the binding nucleic acid molecule and the base length of the blocking nucleic acid molecule is not particularly limited. The base length of the blocking nucleic acid molecule is in the range from 1/1 to 1/20 of the base length of the labeled binding nucleic acid molecule, for example.

As a specific example, in the case where the binding nucleic acid molecule is a nucleic acid molecule including the polynucleotide (a), the blocking nucleic acid molecule may be, for example, a nucleic acid molecule including the following polynucleotide (b). The following polynucleotides (b1) to (b2) are polynucleotides each bind (hybridize) to at least one polynucleotide selected from the group consisting of the polynucleotides (a1) to (a2), for example,
(b) at least one polynucleotide selected from the group consisting of the following polynucleotides (b1) to (b2):
(b1) a polynucleotide consisting of the base sequence of SEQ ID NO: 3, and
(b2) a polynucleotide consisting of a base sequence having at least 90% identity to the base sequence of the polynucleotide (b1).

The polynucleotide (b1) is a polynucleotide consisting of the base sequence of SEQ ID NO: 3.
Complementary strand 1 (SEQ ID NO: 3)
5'-AAAAAAAAAAAAAAAAAAAATAGAGAGTCAGTCCCAACCA-3'

In the polynucleotide (b2), the "identity" may be in any range as long as it allows the polynucleotide (b2) to bind to at least one polynucleotide selected from the group consisting of the polynucleotides (a1) to (a2), for example. The identity is, for example, at least 90%, at least 95%, at least 96%, at least 97%, at least 98%, or at least 99%.

As used herein, "a polynucleotide that hybridizes to a polynucleotide consisting of the base sequence of the polynucleotide (a1)" is, for example, a polynucleotide completely or partially complementary to the polynucleotide (b1). Regarding the "hybridization" and the "stringent condition", for example, reference can be made to the above description.

The blocking nucleic acid molecule may include one base sequence of any one of the polynucleotides (b1) to (b2) or two or more base sequences of the polynucleotides (b1) to (b2), for example. In the latter case, preferably, two or more polynucleotides are linked to form a single-stranded polynucleotide. The two or more polynucleotides may be directly linked or indirectly linked through linkers, for example. Regarding the linker, reference can be made to the above description. The polynucleotides are preferably linked directly or indirectly at their ends. The two or more polynucleotides may be identical to one another or different from one another, for example. The two or more polynucleotides are preferably identical to one another, for example. In the case where there are two or more polynucleotides, the number of polynucleotides is not particularly limited, and is, for example, 2 or more, and specifically, for example, 2 to 20, 2 to 10, 2, or 3. In the case where the binding nucleic acid molecule includes two or more polynucleotides (a), the number of the polynucleotides (b) included in the blocking nucleic acid molecule may be, for example, identical to or different from the number of the polynucleotides (a) included in the binding nucleic acid molecule, and the former is preferred.

The combination of the polynucleotides (a) and the polynucleotides (b) is not particularly limited, and may be, for example, the combination corresponding to the case where the combination of the polynucleotide (a1) and the polynucleotide (b1) is as follows:
the combination of a polynucleotide consisting of the base sequence of SEQ ID NO: 1 of the polynucleotide (a1) and a polynucleotide consisting of the base sequence of SEQ ID NO: 3 of the polynucleotide (b1).

On the carrier, as described above, the blocking nucleic acid molecule is immobilized. The carrier is not particularly limited, and examples thereof include beads, plates, and containers. The material of the carrier is not particularly limited. The carrier may be, for example, a polystyrene carrier, a silica carrier, an agarose carrier, a glass carrier, an acrylic resin carrier, a polyvinyl alcohol resin carrier, or a polycarbonate carrier, and is preferably a polystyrene carrier because it improves analysis sensitivity. The size of the carrier is not particularly limited. The shape of the carrier is not particularly limited. In the case where the carrier is a bead, the shape may be, for example, a sphere such as an oval sphere or a complete sphere.

The blocking nucleic acid molecule may be immobilized on the carrier at either the 5' end or the 3' end, for example, and the 5' end is preferred. The blocking nucleic acid molecule may be directly or indirectly immobilized on the carrier, for example. In the latter case, for example, the blocking nucleic acid molecule is preferably immobilized on the carrier through the linker. Regarding the linker, for example, reference can be made to the above description.

Next, each of the steps is described. The following steps can be performed, for example, in a reaction system that contains the specimen, the binding nucleic acid molecule, the carrier, and the like. The reaction system is preferably a liquid system in which a reaction is performed in liquid, for example. The liquid is not particularly limited, and examples thereof include water, physiological saline, and buffer solutions.

The reaction step is, as described above, a step of causing a specimen, a labeled binding nucleic acid molecule that binds to a target, and a carrier on which a blocking nucleic acid molecule that binds to the binding nucleic acid molecule is immobilized to react. In the reaction step, the order of the reaction of the specimen, the labeled binding nucleic acid molecule, and the carrier is not particularly limited, and may be, for example, as the following orders (x1) to (x3):
(x1) the specimen is caused to react with the labeled binding nucleic acid molecule and then the mixture of the specimen and the labeled binding nucleic acid molecule is caused to react with the carrier;
(x2) the labeled binding nucleic acid molecule is caused to react with the carrier and then the mixture of the labeled binding nucleic acid molecule and the carrier is caused to react with the specimen; and
(x3) the specimen, the binding nucleic acid molecule, and the carrier are caused to react at once.

In the reaction step, the reaction conditions are not particularly limited. The reaction temperature is, for example, 4 to 37°C or 18 to 25°C, and the reaction time is, for example, 1 to 30 minutes or 1 to 5 minutes. In the case where the reaction step is performed in the order (x1) or (x2), the reaction time of the first reaction is, for example, 0 to 0.5 minutes or 0 to 1 minute and the reaction time of the second reaction is, for example, 1 to 30 minutes or 1 to 3 minutes.

Next, the separation step is a step of separating a fraction of the carriers and a fraction of components other than the carriers from each other. The method of separating the fraction of the carriers and the fraction of components other than the carriers from each other is not particularly limited, and can be, for example, a publicly known solid-liquid separation method. Specific examples of the separation method include filtration treatment, membrane separation treatment, centrifugal separation treatment, and precipitation treatment. In the case of the filtration treatment and the membrane separation treatment, the filtration treatment and the membrane separation treatment may be promoted by applying a pressure, for example. In the case where the carrier is a magnetic carrier, the fraction of the magnetic carriers and the fraction of components other than the magnetic carriers may be separated from each other by separating the magnetic carriers using a magnetic body. One of the separation methods may be used alone or two or more of them may be used in combination.

In the present invention, the separation step may further include a collection step of collecting at least one of the fraction of the carriers and the fraction of components other than the carriers. The fraction to be collected can be determined appropriately depending on the fraction to be subjected to the analysis in the analysis step described below, for example.

In the separation step, in the case of separating the fractions by the filtration treatment or the membrane separation treatment, for example, the fraction of the carriers and the fraction of components other than the carriers can be separated by filtrating with a filtering material or a membrane. Furthermore, for example, the fraction which has passed through the filtering material or the membrane can be collected as the fraction of components other than the carriers and the fraction which has not passed through the filtering material or the membrane can be collected as the fraction of the carriers. The pore size of the filtering material and the pore size of the membrane are not particularly limited. For example, the pore size can be determined appropriately depending on the size of the carrier. Specifically, the pore size may be any size as long as it allows the fraction of the carriers and the fraction of components other than the carriers to be separated from each other. Furthermore, in the case of separating the fractions by the centrifugal separation treatment or precipitation treatment, for example, the fraction of the carriers and the fraction of components other than the carriers can be separated from each other by the centrifugation treatment or the precipitation treatment. Moreover, for example, the obtained precipitate can be collected as the fraction of the carriers, and the fraction other than the precipitate can be collected as the fraction of components other than the carriers. In the separation step, in the case of collecting the fraction of components other than the carriers, for example, the whole or a part of the fraction of components other than the carriers may be collected.

The analysis step is a step of detecting a label of the labeled binding nucleic acid molecule in at least one of the fraction of the carriers and the fraction of components other than the carriers to analyze a target in the specimen. In the analysis step, the fraction to be subjected to the analysis is, for example, either the fraction of the carriers or the fraction of components other than the carriers or both of the fractions, and is preferably the fraction of components other than the carriers because it improves analysis sensitivity. In the case of using both of the fractions, preferably, the fraction of the carriers and the fraction of components other than the carriers are collected separately and each of the fractions is subjected to the analysis.

In the analysis step, the detection of the label is not particularly limited, and can be determined appropriately depending on the kind of the label, for example. As a specific example, in the case where the label is an enzyme, the detection of the label is, for example, the detection of an enzymatic reaction. More specifically, the detection of the label is the detection of the optical signals, electrical signals, and the like caused by the enzymatic reaction. As to the optical signal, for example, the light emission, fluorescence, color development, and the like may be detected by a visual observation or the light emission intensity, fluorescence intensity, absorbance, reflectance, and the like as signals may be detected by an optical method. The electrical signal may be, for example, a current. The electrical signal can be detected, for example, by an electrical method. The enzymatic reaction is not particularly limited, and may be, for example, a redox reaction. In the case where the label is a fluorescent substance, the detection of the label is, for example, the detection of the fluorescence caused by the fluorescent substance. In the case where the label is an isotope, the detection of the label is, for example, the detection of the radiation signals caused by the isotope. As to the radiation signal, for example, the fluorescence action, ionization action, and the like of α-rays, β-rays, γ-rays, positron, and X-rays may be detected by an optical or electrical method.

In the case of detecting the enzymatic reaction in the analysis step, preferably, the enzymatic reaction is detected in the presence of a substrate for the enzyme. The substrate is not particularly limited and can be determined appropriately depending on the kind of the enzyme, for example. As a specific example, in the case where the enzyme is luciferase, examples of the substrate include luciferin and coelenterazine.

The analysis method of the present invention may further include a calculation step of calculating the concentration of the target in the specimen from the detection result obtained in the analysis step. Examples of the detection result include optical signals and electrical signals. In the calculation step, the concentration of the target can be calculated based on the detection result and the correlation between the detection result and the concentration of the target in the specimen, for example. The correlation can be obtained, with respect to a standard specimen in which the concentration of the target is known, by plotting the detection result obtained by the analysis method of the present invention and the concentration of the target in the standard specimen, for example. The standard specimen is preferably dilution series of the target. The calculation of the concentration in this manner makes it possible to perform a highly reliable quantitative analysis.

In the analysis method of the present invention, the structural unit of each of the binding nucleic acid molecule and the blocking nucleic acid molecule is, for example, a nucleotide residue, and examples of the nucleotide residue include deoxyribonucleotide residues and ribonucleotide residues. The polynucleotide may, for example, DNA consisting of deoxyribonucleotide residues or DNA containing deoxyribonucleotide residues and ribonucleotide residues. The polynucleotide may further contain non-nucleotide residues. In the latter case, for example, "one to several" is not particularly limited and is, for example, 1 to 91, 1 to 30, 1 to 15, 1 to 7, 1 to 3, 1, or 2 in the polynucleotide. In the present invention, for example, the numerical range regarding the number of bases, the number of sequences, and the like discloses all the positive integers falling within that range. That is, for example, the description "1 to 5 bases" discloses all of "1, 2, 3, 4, and 5 bases" (hereinafter, the same applies).

The polynucleotide may contain a modified base. The modified base is not particularly limited, and may be, for example, a base obtained by modifying a natural base (inartificial base). The modified base preferably has the same function as the natural base. The natural base is not particularly limited, and examples thereof include a purine base having a purine skeleton and a pyrimidine base having a pyrimidine skeleton. The purine base is not particularly limited, and examples thereof include adenine (a) and guanine (g). The pyrimidine base is not particularly limited, and examples thereof include cytosine (c), thymine (t), and uracil (u). The modification site in the base is not particularly limited. In the case where the base is a purine base, the modification site in the purine base may be, for example, the 7-position and the 8-position of the purine skeleton. In the case where the base is a pyrimidine base, the modification site in the pyrimidine base may be, for example, the 5-position and the 6-position of the pyrimidine skeleton. In the case where "=O" is bound to the 4-position carbon and a group other than "-CH₃" or "-H" is bound to 5-position carbon in the pyrimidine skeleton, the base can be referred to as modified uracil or modified thymine.

The modifying group of the modified base is not particularly limited, and examples thereof include methyl groups, fluoro groups, amino groups, thio groups, benzylaminocarbonyl groups represented by the following chemical formula (1), and tryptaminocarbonyl groups and isobutylaminocarbonyl groups represented by the following chemical formula (2).

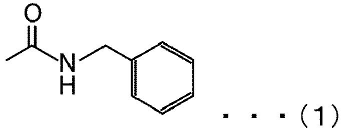

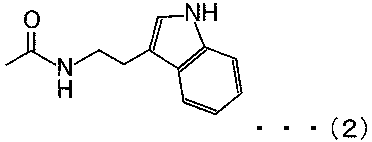

The modified base is not particularly limited, and examples thereof include modified adenine in which adenine is modified, modified thymine in which thymine is modified, modified guanine in which guanine is modified, modified cytosine in which cytosine is modified, and modified uracil in which uracil is modified, and the modified thymine, modified uracil, and the modified cytosine are preferred.

A specific example of the modified adenine may be 7'-deazaadenine.

A specific example of the modified guanine may be 7'-deazaguanine.

Specific examples of the modified thymine include 5'-benzylaminocarbonyl thymine, 5'-tryptaminocarbonyl thymine, and 5'-isobutylaminocarbonyl thymine.

Specific examples of the modified uracil include 5'-benzylaminocarbonyl uracil (BndU), 5'-tryptaminocarbonyl uracil (TrpdU), and 5'-isobutylaminocarbonyl uracil.

The polynucleotide may contain one of the modified bases or two or more of the modified bases, for example.

The number of the modified bases is not particularly limited. In the polynucleotide, the number of the modified bases is not particularly limited. The number of the modified bases in the polynucleotide is, for example, 1 to 100, 1 to 90, 1 to 80, 1 to 70, 1 to 60, 1 to 40, 1 to 20, 1 to 10, or 1 to 5. All the bases of the polynucleotide may be the modified bases. The number of the modified bases may be the number of the modified bases of one kind or the total number of the modified bases of two or more kinds, for example.

In the case where the polynucleotide contains the modified base, the proportion of the modified base is not particularly limited. The proportion of the modified base is at least 1/100, at least 1/40, at least 1/20, at least 1/10, at least 1/4, or at least 1/3 of all the bases of the polynucleotide, for example. While the proportion of the modified base is indicated by a fraction, the number of all the bases and the number of the modified bases satisfying the fraction are each positive integer.

The binding nucleic acid molecule and the blocking nucleic acid molecule each may contain a modified nucleotide, for example. The modified nucleotide may be a nucleotide containing the aforementioned modified base or a nucleotide containing modified sugar obtained by modifying a sugar residue, or a nucleotide containing the modified base and the modified sugar.

The sugar residue is not particularly limited, and examples thereof include deoxyribose residues and ribose residues. The modification site in the sugar residue is not particularly limited, and may be, for example, the 2'-position or the 4'-position of the sugar residue. Either one of or both of the 2'-position and the 4'-position may be modified. Examples of the modifying group of the modified sugar include methyl groups, fluoro groups, amino groups, and thio groups.

In the case where the base is a pyrimidine base in the modified nucleotide residue, for example, it is preferable that the 2'-position and/or the 4'-position of the sugar residue is modified. Specific examples of the modified nucleotide residue include, 2'-methylated-uracil nucleotide residues, 2'-methylated-cytosine nucleotide residues, 2'-fluorinated-uracil nucleotide residues, 2'-fluorinated-cytosine nucleotide residues, 2'- aminated-uracil nucleotide residues, 2'-aminated-cytosine nucleotide residues, 2'- thiolated-uracil nucleotide residues, and 2'-thiolated-cytosine nucleotide residues each obtained by modifying the 2'-position of deoxyribose residues or ribose residues.

The number of modified nucleotides is not particularly limited and is, for example, 1 to 100, 1 to 90, 1 to 80, or 1 to 70 in the polynucleotide. Furthermore, the number of the modified nucleotides in the full-length of the binding nucleic acid molecule and the blocking nucleic acid molecule each including the polynucleotide is not particularly limited, and is specifically the same range as described above, for example.

The binding nucleic acid molecule and the blocking nucleic acid molecule may contain one to several artificial nucleic acid monomer residues, for example. "one to several" is not particularly limited and is, for example, 1 to 100, 1 to 50, 1 to 30, or 1 to 10 in the polynucleotide. Examples of the artificial nucleic acid monomer residue include peptide nucleic acids (PNAs), locked nucleic acids (LNAs), and 2'-0,4'-C-ethylenebridged nucleic acids (ENAs). As to the nucleic acids in the monomer residues, for example, reference can be made to the above description.

The binding nucleic acid molecule and the blocking nucleic acid molecule may further include, for example, an additional sequence. The additional sequence is bound to at least one of the end of the binding nucleic acid molecule which is not labeled and the end of the binding nucleic acid molecule on which the blocking nucleic acid molecule is not immobilized, for example. The additional sequence is not particularly limited. The length of the additional sequence is not particularly limited, and is, for example, 1 to 200-mer, 1 to 50-mer, 1 to 25-mer, or 18 to 24-mer. The structural unit of the additional sequence is, for example, a nucleotide residue. Examples of the nucleotide residue include deoxyribonucleotide residues and ribonucleotide residues. The additional sequence is not particularly limited, and examples thereof include polynucleotides such as DNA consisting of deoxyribonucleotide residues and DNA containing ribonucleotide residues. Specific examples of the additional sequence include poly dT, and poly dA.

In the analysis method of the present invention, the method for producing the binding nucleic acid molecule and the blocking nucleic acid molecule is not particularly limited. The binding nucleic acid molecule and the blocking nucleic acid molecule can be synthesized by a nucleic acid synthesis method utilizing chemical synthesis, a genetic engineering technique, and a known method, for example. Furthermore, the binding nucleic acid molecule can be also obtained by a so-called SELEX method using, for example, a target.

### <Target analysis kit >

The use of a target analysis kit according to the methods of the present invention is, as described above, characterized in that it includes: a labeled binding nucleic acid molecule that binds to a target; and a carrier on which a blocking nucleic acid molecule that binds to the labeled binding nucleic acid molecule is immobilized, wherein the kit is for use in the target analysis method of the present invention. The analysis kit is characterized in that it includes the labeled binding nucleic acid molecule and the carrier, wherein the kit is for use in the analysis method of the present invention. Other configurations and conditions are by no means limited. Regarding the analysis kit, for example, reference can be made to the description as to the analysis method of the present invention. According to the target analysis kit, for example, the analysis method of the present invention can be performed easily. In addition, according to the analysis kit, for example, a target can be analyzed with high analysis sensitivity in a very short time of about 3 minutes. Furthermore, according to the analysis kit, for example, a target can be analyzed with high analysis accuracy, for example, because nonspecific binding of the binding nucleic acid molecule can be suppressed.

In the analysis kit for example, the binding nucleic acid molecule and the carrier may be contained in separate containers or may be contained in the same container in a mixed state or an unmixed state. In the latter case, the analysis kit can also be referred to as, for example, an analysis reagent.

The analysis kit may further include other components, for example. Examples of the component include the substrates, reagents such as buffer solutions, and instruction manuals. The reagent may be contained in a separate container from the binding nucleic acid molecule and the carrier or in the same container with the binding nucleic acid molecule and the carrier in a mixed state or an unmixed state, for example. The substrate is contained in a separate container from the binding nucleic acid molecule and the carrier, for example.

### <Peanut-binding nucleic acid molecule >

The peanut-binding nucleic acid molecule of the present disclosure is characterized in that it includes at least one polynucleotide (hereinafter, also referred to as "polynucleotide (c)") selected from the group consisting of the following polynucleotides (c1) to (c4).
(c1) a polynucleotide consisting of the base sequence of SEQ ID No.: 1; and
(c2) a polynucleotide that binds to a peanut allergen and is consisting of a base sequence having at least 90% identity to the base sequence of the polynucleotide (c1).

Regarding the peanut-binding nucleic acid molecule of the present disclosure for example, reference can be made to the description as to the target analysis method of the present invention. According to the peanut-binding nucleic acid molecule of the present disclosure for example, peanut allergens can be analyzed.

The polynucleotide (c1) is a polynucleotide consisting of any one of the base sequences of SEQ ID No.: 1.

Regarding the polynucleotides (c1) to (c2), for example, reference can be made to the description as to the polynucleotides (a1) to (a2) by replacing "(a1)" with "(c1)", "(a2)" with "(c2)", respectively.

The peanut-binding nucleic acid molecule of the present disclosure may be, for example, a double strand. In the case of a double strand, for example, one of single-stranded polynucleotides includes at least one polynucleotide selected from the group consisting of the polynucleotides (c1) to (c2) and the other of the single-stranded polynucleotides is not limited. The other of the single-stranded polynucleotides may be, for example, a polynucleotide including a base sequence complementary to at least one polynucleotide selected from the group consisting of the polynucleotides (c1) to (c2).

The base sequence complementary to at least one polynucleotide selected from the group consisting of the polynucleotides (c1) to (c2) may be, for example, the following polynucleotide (b). The following polynucleotides (b2) are polynucleotides which each bind (complementary) to at least one polynucleotide selected from the group consisting of the polynucleotides (c1) to (c2), for example.
(b) at least one polynucleotide selected from the group consisting of the following polynucleotides (b1) to (b2):
(b1) a polynucleotide consisting of the base sequence of SEQ ID NO: 3; and
(b2) a polynucleotide consisting of a base sequence having at least 90% identity to the base sequence of the polynucleotide (b1).

The combination of the polynucleotides (c) and the polynucleotides (b) is not particularly limited, and may be, for example, the combination corresponding to the case where the combination of the polynucleotide (c1) and the polynucleotide (b1) is as follows:
the combination of a polynucleotide consisting of the base sequence of SEQ ID NO: 1 of the polynucleotide (c1) and a polynucleotide consisting of the base sequence of SEQ ID NO: 3 of the polynucleotide (b1).

### Examples

The examples of the present invention are described below. The present invention, however, is not limited by the following examples. Commercially available reagents are used according to the protocols of the reagents, unless otherwise noted.

### [Example 1]

It was examined whether a target such as a peanut can be analyzed quickly by the analysis method and the analysis kit of the present invention.

### (1) Specimen

Commercially available peanuts (seeds) were ground with a mill. 5 g of the thus-obtained powder was mixed with 20 ml of a SB1T buffer solution, and the resultant mixed solution was shaken overnight (for about 16 hours, the same applies hereinafter) using a shaker at 90 to 110 rpm at room temperature (around 25°C, the same applies hereinafter). The SB IT buffer solution had the following composition: 40 mmol/l HEPES (pH 7.5), 125 mmol/l NaCl, 25 mmol/l KCl, 1 mmol/l MgCl₂, and 0.05 (v/v)% Tween® 20.

The mixed solution after being shaken was centrifuged overnight at 10000 g at room temperature. Then, the supernatant was collected, and the supernatant was filtered through a filter (pore size: 0.8 µm) to prepare a peanut extract 1. Then, the peanut protein concentration in the peanut extract 1 was quantified using a protein concentration measurement kit (Protein Assay reagent, Bio-Rad).

### (2) Analysis

A binding nucleic acid molecule solution containing labeled binding nucleic acid molecules that bind to peanut allergen was prepared by mixing a solution containing a polynucleotide (peanut aptamer) consisting of the base sequence of SEQ ID NO: 1 whose 5' end is biotinylated and a solution containing streptavidin-labeled luciferase (Streptavidin-Lucia, Invivogen). The labeled binding nucleic acid molecules were prepared by immobilizing 400 pmol of the binding nucleic acid molecules per 100 pmol of the streptavidin. The concentration of the binding nucleic acid molecule in the binding nucleic acid molecule solution was 1 pmol/l. Carriers on which blocking nucleic acid molecules are immobilized were prepared by mixing a polynucleotide consisting of the base sequence of SEQ ID NO: 3 whose 5' end is biotinylated and streptavidin-labeled beads (Invitrogen), shaking the thus-obtained mixture for 30 minutes at room temperature, and then washing the resultant with the SB1T buffer solution. The carriers on which the blocking nucleic acid molecules are immobilized (immobilized blocking nucleic acid molecules) were prepared by immobilizing 500 pmol of the blocking nucleic acid molecules per mg of the beads. After the washing, the beads were suspended in the SB1T buffer solution so as to have a concentration of 40 mg/ml to prepare a bead solution. It is to be noted that the beads were magnetic beads.

Peanut diluted solutions were prepared by diluting the peanut extract 1 with a SB1T buffer solution so as to achieve predetermined peanut protein concentrations (0.0004, 0.002, 0.01, 0.05, 0.25, 1.25, and 6.25 ppm) in a reaction solution obtained after being mixed with the binding nucleic acid molecules and the beads. Then, a bead diluted solution was prepared by adding 4 µl of the bead solution and 16 µl of the SB1T buffer solution to each well of a U-bottom plate. Furthermore, to each well of another U-bottom plate, 25 µl of the binding nucleic acid molecule solution was added and 25 µl of each peanut diluted solution was further added. After the addition, each well was incubated for 1 minute at room temperature. Then, 20 µl of the bead diluted solution was added to each well after being incubated, and the resultant mixed solution was shaken for 2 minutes at 1000 rpm at room temperature using the shaker.

Subsequently, the plate was set on a magnetic bead separation plate, and a fraction of the magnetic beads and a fraction of components other than the magnetic beads were separated. After the separation, 30 µl of the supernatant was collected from each well and added to each well of a measurement plate (white half plate, Greiner Bio One). Then, 30 µl of a substrate solution was added to the supernatant, and the resultant mixed solution was pipetted.
Thereafter, the amount of light emission in each well was measured using a plate reader (Infinite M1000 Pro, TECAN). In the case where the enzyme was luciferase, Quanti-Luc (product name, invivogen) was used as the substrate solution. The amount of light emission was measured in the same manner as described above except that a polynucleotide consisting of the base sequence of SEQ ID NO: 2 (not part of the invention) were used instead of a polynucleotide consisting of the base sequence of SEQ ID NO: 1, streptavidin-labeled alkaline phosphatase (GE Healthcare Biosciences) was used instead of streptavidin-labeled luciferase, a polynucleotide consisting of the base sequence of SEQ ID NO: 4 (not part of the invention) was used instead of a polynucleotide consisting of the base sequence of SEQ ID NO: 3, the predetermined concentrations were changed to 0, 0.25, 1, 4, 16, and 64 ppm, and CDP-star (Emerald II) (product name, Roche) was used as the substrate solution.

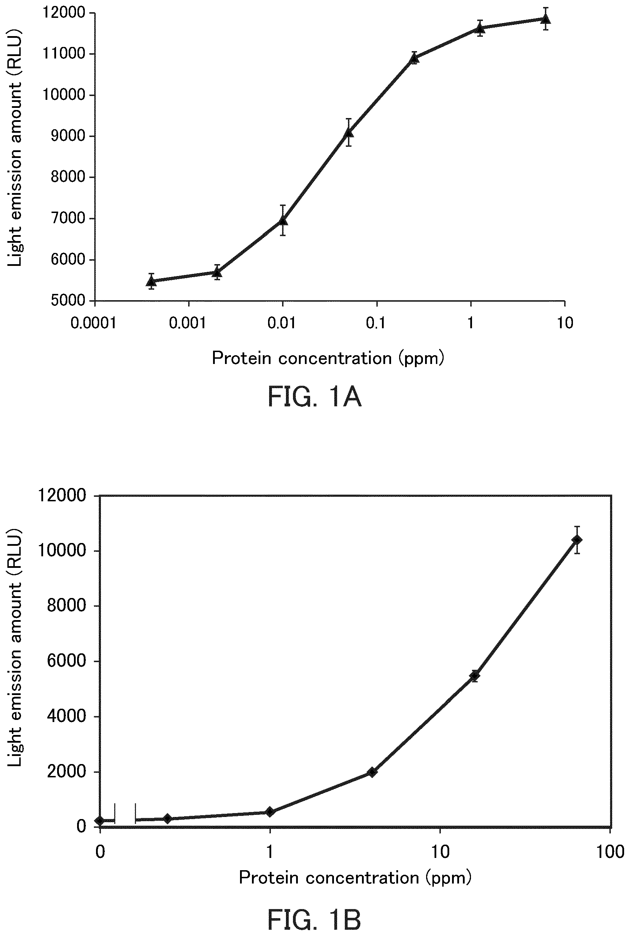

The results thereof are shown in FIGs. 1A and 1B. FIGs. 1A and 1B are graphs each showing the amount of light emission. FIG. 1A shows the result obtained in the case where the enzyme is luciferase and FIG. 1B shows the result obtained in the case where the enzyme is alkaline phosphatase. In each of FIGs. 1A and 1B, the horizontal axis indicates the peanut protein concentration and the vertical axis indicates the amount of light emission. As can be seen in FIGs. 1A and 1B, the amount of light emission increased in a peanut protein concentration dependent manner in either case of using alkaline phosphatase or luciferase as the label. This demonstrates that even a target having a peanut protein concentration of about 0.001 ppm can be analyzed with excellent analysis sensitivity in the case where luciferase was used as the label. Furthermore, the analysis could be performed with the incubation time in total of 3 minutes, which demonstrates that a target such as a peanut allergen can be analyzed quickly by the analysis method and the analysis kit of the present invention.

### [Example 2]

It was examined whether peanuts in food can be analyzed by the analysis method and the analysis kit of the present invention.

### (1) Specimen

A cookie extract, a chocolate extract, and a biscuit extract were prepared in the same manner as in Example 1 (1) except that commercially available cookies (mini Butter Cookies, Ito Bicuits Co., LTD.), chocolates (DARS milk chocolate, MORINAGA & CO., LTD.), and biscuits (Sand Biscuit, East pigeon Co,. LTD.) were used instead of the peanuts, and the mixed solution after being mixed with the SB1T buffer solution were shaken for 1 minute. Then, the protein concentration in each of the extracts was quantified in the same manner as in Example 1 (1).

### (2) Analysis

The amount of light emission was measured in the same manner as in Example 1 (2) except that the cookie extract, the chocolate extract, and the biscuit extract were used instead of the peanut extract 1, each of the extracts was added so as to achieve the extract-derived protein concentration of 10 ppm in a reaction solution obtained after being mixed with the binding nucleic acid molecules and the beads, and the peanut extract 1 was further added so as to achieve predetermined peanut protein concentrations (0.01, 0.05, 0.25, 1.25, and 6.25 ppm) in the reaction solution.

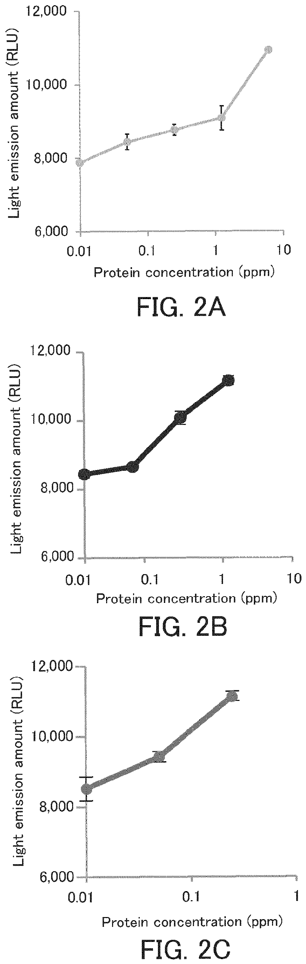

The results thereof are shown in FIGs. 2A to 2C. FIGs. 2A to 2C are graphs each showing the amount of light emission. FIG. 2A shows the result obtained with the cookie extract, FIG. 2B shows the result obtained with the chocolate extract, and FIG. 2C shows the result obtained with the biscuit extract. In each of FIGs. 2A to 2C, the horizontal axis indicates the peanut protein concentration and the vertical axis indicates the amount of light emission. As can be seen in FIGs. 2A to 2C, regardless of the food used, the amount of light emission increased in a peanut protein concentration dependent manner. This demonstrates that peanuts in food can be analyzed by the analysis method and the analysis kit of the present invention even in a case where the food containing many admixtures and the like is used.

### [Example 3]

It was examined whether peanuts can be analyzed by the analysis method and the analysis kit of the present invention using different carriers.

### (1) Specimen

The peanut extract 1 was prepared in the same manner as in Example 1 (1).

### (2) Analysis

The amount of light emission was measured in the same manner as in Example 1 (2) except that streptavidin-labeled agarose beads (Invitrogen) or streptavidin-labeled resin (polystyrene) beads (SA-resin beads, Bio-rad) were used instead of the streptavidin-labeled beads, and the luciferase was used as the enzyme.

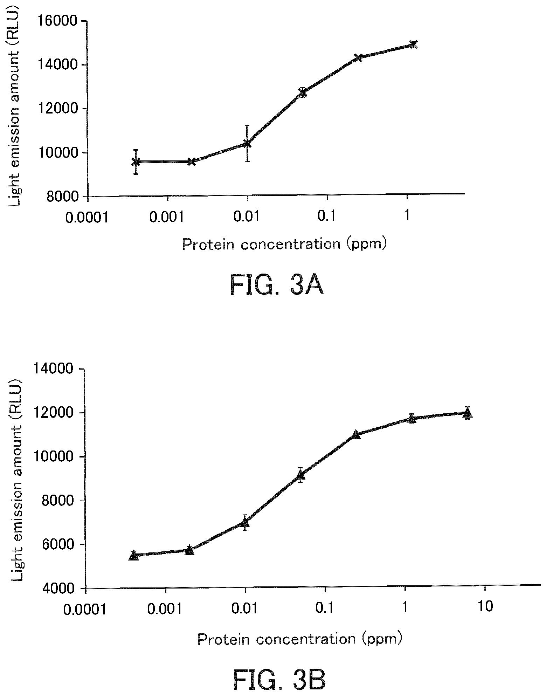

The results thereof are shown in FIGs. 3A and 3B. FIGs. 3A and 3B are graphs each showing the amount of light emission. FIG. 3A shows the result obtained with the agarose beads and FIG. 3B shows the result obtained with the resin beads. In each of FIGs. 3A and 3B, the horizontal axis indicates the peanut protein concentration and the vertical axis indicates the amount of light emission. As can be seen in FIGs. 3A and 3B, the amount of light emission increased in a peanut protein concentration dependent manner in either case of using the streptavidin-labeled agarose beads or the streptavidin-labeled resin (polystyrene) beads. Furthermore, the protein could be detected in a wider range of concentrations in the case of using the resin beads. This demonstrates that peanuts can be analyzed with either of the carriers by the analysis method and the analysis kit of the present invention.

### [Example 4]

It was examined whether peanuts can be analyzed and nonspecific binding is reduced by the analysis method and the analysis kit of the present invention.

### (1) Specimen

The peanut extract 1 was prepared and the protein concentration was quantified in the same manner as in Example 1 (1). Furthermore, a soybean extract was prepared in the same manner as in Example 1 (1) except that commercially available soybeans (seeds) were used instead of the peanuts, and the protein concentration was quantified in the same manner as in Example 1 (1). It is to be noted that soybeans are known to contain proteins having high homology to peanut allergen.

### (2) Analysis

The binding nucleic acid molecule solution was prepared in the same manner as in Example 1 (2). Furthermore, a bead solution was prepared in the same manner as in Example 1 (2) except that the streptavidin-labeled resin beads were used as the streptavidin-labeled beads and the luciferase was used as the enzyme. Peanut diluted solutions were prepared by diluting the peanut extract 1 with the SB1T buffer solution so as to achieve predetermined protein concentrations (0, 1.25, 5, 20, and 80 ppm) in a reaction solution obtained after being mixed with the binding nucleic acid molecules and the beads. Subsequently, a bead diluted solution was prepared by adding 40 µl of the bead solution and 160 µl of the SB1T buffer solution to a tube. To another tube, 250 µl of the binding nucleic acid molecule solution was added, and 250 µl of each peanut diluted solution was further added. After the addition, the tube was agitated. Further, 200 µl of the bead diluted solution was added to the tube. Thereafter, the tube was agitated, and then incubated at room temperature for 1 minute.

The reaction solution after the incubation was filtered using a 5 ml syringe and a filter (pore size: 0.45 µm), and the filtrate was collected in a measurement container to which 800 µl of the substrate solution had been added beforehand. After the collection, the measurement container was agitated, and incubated for 30 seconds to 1 minute. Then, using a luminometer (Clean-Trace (trademark), 3M Co.), the amount of light emission in each measurement sample in the measurement container was measured. Furthermore, the amount of light emission was measured in the same manner as described above except that the soybean diluted solution prepared by diluting the soybean extract with the SB1T buffer solution so as to achieve a protein concentration of 80 ppm in a reaction solution obtained after being mixed with the binding nucleic acid molecules and the beads was used instead of the peanut diluted solution.

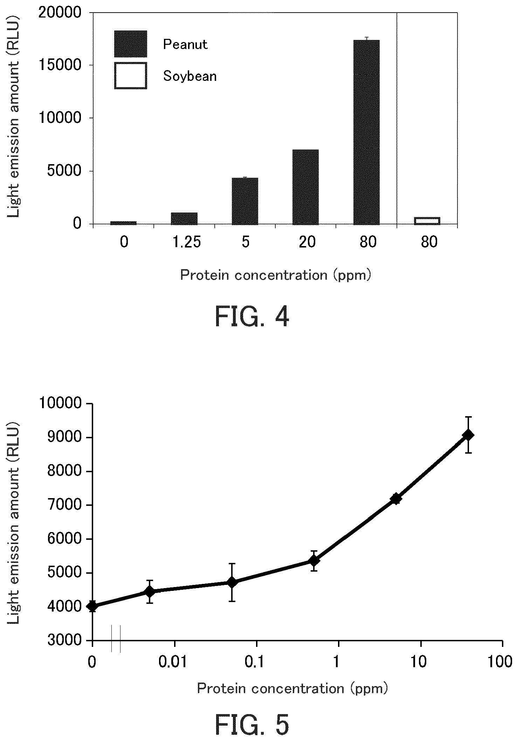

The results thereof are shown in FIG. 4. FIG. 4 is a graph showing the amount of light emission. In FIG. 4, the horizontal axis indicates the peanut or soybean protein concentration and the vertical axis indicates the amount of light emission. As can be seen in FIG. 4, in the case where the peanut aptamers were used as the binding nucleic acid molecules, the amount of light emission increased in a peanut extract 1-derived peanut protein concentration dependent manner. On the other hand, in the case where the soybean extract was used, the amount of light emission was equivalent to the background and the binding nucleic acid molecules did not bind to soybean proteins. This demonstrates that targets such as peanuts can be analyzed and nonspecific binding is reduced by the analysis method and the analysis kit of the present invention.

### [Example 5]

It was examined whether peanuts can be analyzed by the analysis method and the analysis kit of the present invention.

### (1) Specimen

The mixture after being shaken was prepared in the same manner as in Example 1 (1) except that the peanut powder was mixed with the SB1T buffer solution and then the resultant mixed solution was shaken for 1 minute. The mixture after being shaken was centrifuged for 30 minutes at 10000 g at room temperature. Then, the supernatant was collected, and the supernatant was filtered through a filter (pore size: 0.8 µm) to prepare a peanut extract 2. Then, the peanut protein concentration in the peanut extract 2 was quantified in the same manner as in Example 1 (1).

Samples were prepared by diluting the peanut extract 2 with the SB1T buffer solution so as to achieve predetermined peanut protein concentrations (0, 1, 10, 100, 1000, and 7700 ppm). 100 µl of each sample was applied to aluminum foil. Thereafter, the aluminum foil was wiped with a cotton swab. The cotton swab was brought into contact with 400 µl of the SB1T buffer solution. The resultant mixed solution was incubated at room temperature for 1 minute, thereby extracting the target held by the cotton swab was extracted. Thus, an extract was obtained. 25 µl of the binding nucleic acid molecule solution was added to 25 µl of the extract so that the amount of the binding nucleic acid molecules derived from the labeled binding nucleic acid molecules was 1 pmol. The resultant mixed solution was stirred at room temperature for 1 minute, thereby causing the labeled binding nucleic acid molecules to bind to the peanut allergen as the target. To this mixed solution, 4 µl of the bead solution (the blocking nucleic acid molecules: 40 pmol) was further added so that the resultant mixed solution contained the same amount of the blocking nucleic acid molecules (10 µmol/l) as the binding nucleic acid molecules derived from the labeled binding nucleic acid molecules. The resultant mixed solution was stirred at room temperature for 4 minutes, thereby causing the immobilized blocking nucleic acid molecules to bind to the binding nucleic acid molecules in the labeled binding nucleic acid molecules not bound to the target.

Next, the resultant mixed solution was set in a magnetic holder to separate the mixed solution into a fraction of the magnetic beads and a fraction of components other than the magnetic beads, and the latter fraction was collected. Then, 30 µl of a substrate solution (Quanti-Luc (trademark), Invivogen) was added to 30 µl of the supernatant, and the resultant mixed solution was pipetted. Thereafter, the amount of light emission in each sample was measured using a plate reader (Infinite M1000 Pro, TECAN).

The results thereof are shown in FIG. 5. FIG. 5 is a graph showing the amount of light emission. In FIG. 5, the horizontal axis indicates the peanut protein concentration in the mixed solution obtained by mixing the extract with the binding nucleic acid molecule solution, and the vertical axis indicates the amount of light emission. As can be seen in FIG. 5, the amount of light emission increased in a peanut protein concentration dependent manner. This demonstrates that targets such as peanuts can be analyzed by the analysis method and the analysis kit of the present invention.

### [Example 6]

It was examined whether peanuts can be analyzed by the analysis method and the analysis kit of the present invention.

First, a peanut extract 2, a binding nucleic acid molecule solution, and a bead solution were prepared in the same manner as in Example 1 or 3 except that streptavidin-labeled resin beads (Pierce (trademark) Streptavidin Plus UltraLink (trademark) Resin, PIERCE) were used instead of the magnetic beads.

Samples were prepared by diluting the peanut extract 2 with the SB1T buffer solution so as to achieve predetermined peanut protein concentrations (0, 0.002, 0.01, 0.05, 0.25, 1.25, and 6.25 ppm). 25 µl of the binding nucleic acid molecule solution was added to 25 µl of each sample so that the amount of the binding nucleic acid molecules derived from the labeled binding nucleic acid molecules was 1 pmol. The resultant mixed solution was stirred at room temperature for 1 minute, thereby causing the labeled binding nucleic acid molecules to bind to the peanut allergen as the target. To this mixed solution, 10 µl of the bead solution (the blocking nucleic acid molecules: 40 pmol) was further added so that the resultant mixed solution contained the same amount of the blocking nucleic acid molecules (4 µmol/l) as the binding nucleic acid molecules derived from the labeled binding nucleic acid molecules. The resultant mixed solution was stirred at room temperature for 4 minutes, thereby causing the immobilized blocking nucleic acid molecules to bind to the binding nucleic acid molecules in the labeled binding nucleic acid molecules not bound to the target protein. The resultant mixed solution was subjected to centrifugal filtration using a filter plate (pore size: 0.22 µm, MultiScreen®, Millipore Corporation). 30 µl of the substrate solution was added to 30 µl of the thus-obtained filtrate, and the resultant mixed solution was pipetted. Thereafter, the amount of light emission in each sample was measured using the plate reader.

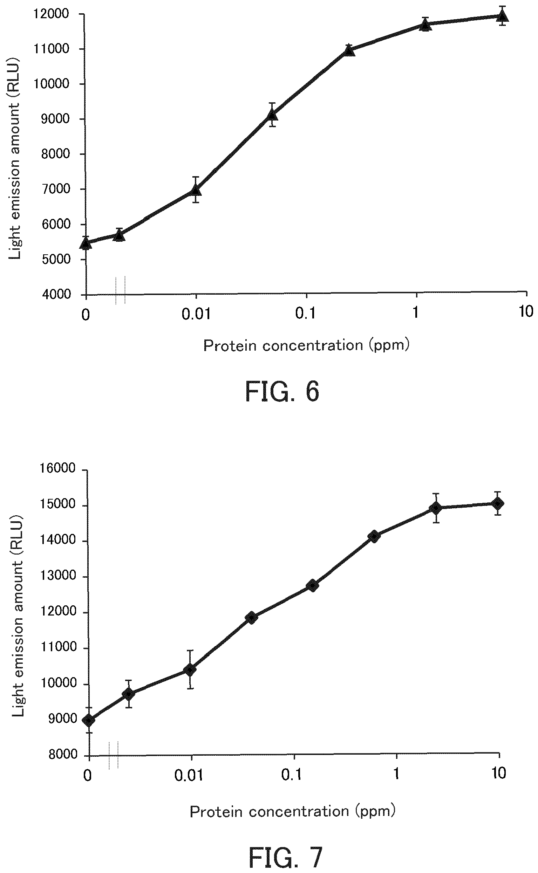

The results thereof are shown in FIG. 6. FIG. 6 is a graph showing the amount of light emission. In FIG. 6, the horizontal axis indicates the peanut protein concentration, and the vertical axis indicates the amount of light emission. As can be seen in FIG. 6, the amount of light emission increased in a peanut protein concentration dependent manner. This demonstrates that targets such as peanuts can be analyzed by the analysis method and the analysis kit of the present invention.

### [Example 7]

It was examined whether peanuts can be analyzed by the analysis method and the analysis kit of the present invention.

First, a peanut extract 2, a binding nucleic acid molecule solution, and a bead solution were prepared in the same manner as in Example 1 or 3.

Samples were prepared by diluting the peanut extract 2 with the SB1T buffer solution so as to achieve predetermined peanut protein concentrations (0, 0.002, 0.01, 0.05, 0.25, 1.25, and 6.25). 25 µl of the bead solution was added to 25 µl of each sample so that the amount of the blocking nucleic acid molecules derived from the immobilized blocking nucleic acid molecules was 1 pmol, and the resultant mixed solution was stirred at room temperature for 1 minute. To this mixed solution, 4 µl of the binding nucleic acid molecule solution (the binding nucleic acid molecules: 40 pmol) was further added so that the resultant mixed solution contained the same amount of the binding nucleic acid molecules (10 µmol/l) as the blocking nucleic acid molecules derived from the immobilized blocking nucleic acid molecules. The resultant mixed solution was stirred at room temperature for 4 minutes, thereby causing the labeled binding nucleic acid molecules to bind to the peanut allergen as the target and also causing the immobilized blocking nucleic acid molecules to bind to the binding nucleic acid molecules in the labeled binding nucleic acid molecules not bound to the target protein.

Next, the resultant mixed solution was set in a magnetic holder to separate the mixed solution into a fraction of the magnetic beads and a fraction of components other than the magnetic beads, and the latter fraction was collected. Then, 30 µl of the substrate solution was added to 3 µl of the supernatant, and the resultant mixed solution was pipetted. Thereafter, the amount of light emission in each sample was measured using the plate reader.

The results thereof are shown in FIG. 7. FIG. 7 is a graph showing the amount of light emission. In FIG. 7, the horizontal axis indicates the peanut protein concentration, and the vertical axis indicates the amount of light emission. As can be seen in FIG. 7, the amount of light emission increased in a peanut protein concentration dependent manner. This demonstrates that targets such as peanuts can be analyzed by the analysis method and the analysis kit of the present invention.

## Claims

1. A target analysis method comprising:
causing a specimen, a labeled binding nucleic acid molecule that binds to a target, and a carrier on which a blocking nucleic acid molecule that binds to the labeled binding nucleic acid molecule is immobilized to react;
separating a fraction of the carrier and a fraction of components other than the carrier from each other; and
detecting a label of the labeled binding nucleic acid molecule in at least one of the fraction of the carrier or the fraction of components other than the carrier to analyze a target in the specimen,
wherein
the labeled binding nucleic acid molecule includes the following polynucleotide (a):
(a) the following polynucleotide (a1) or (a2):
(a1) a polynucleotide consisting of a base sequence of SEQ ID NO: 1, and
(a2) a polynucleotide that binds to a peanut allergen and is consisting of a base sequence having at least 90% identity to the base sequence of the polynucleotide (a1).

2. The method according to claim 1, comprising:
causing the specimen to react with the labeled binding nucleic acid molecule; and
causing a mixture of the specimen and the labeled binding nucleic acid molecule to react with the carrier.

3. The method according to claim 1 or 2, wherein
the blocking nucleic acid molecule is a nucleic acid molecule that includes a base sequence complementary to the labeled binding nucleic acid molecule.

4. The method according to any one of claims 1 to 3, wherein
the blocking nucleic acid molecule is a nucleic acid molecule that includes a base sequence complementary to a base sequence that binds to a target in the labeled binding nucleic acid molecule.

5. The method according to any one of claims 1 to 4, wherein
the blocking nucleic acid molecule is a nucleic acid molecule that includes a polynucleotide consisting of a base sequence having complementarity in a range from 5% to 100% with respect to a base sequence of the labeled binding nucleic acid molecule.

6. The method according to any one of claims 1 to 5, wherein
the blocking nucleic acid molecule includes the following polynucleotide (b):
(b) the following polynucleotide (b1) or (b2):
(b1) a polynucleotide consisting of a base sequence of SEQ ID NO: 3, and
(b2) a polynucleotide consisting of a base sequence having at least 90% identity to the base sequence of the polynucleotide (b1).

7. The method according to any one of claims 1 to 6, wherein
the carrier is a bead, preferably a polystyrene bead or a magnetic bead.

8. The method according to claim 7, wherein
the fraction of the magnetic bead and the fraction of components other than the magnetic bead are separated from each other by separating the magnetic bead using a magnetic body.

9. The method according to any one of claims 1 to 8, wherein
the label is an enzyme, preferably luciferase, and
an enzymatic reaction of the labeled binding nucleic acid molecule in at least one of the fraction of the carrier or the fraction of components other than the carrier is detected.

10. The method according to claim 9, wherein
the enzymatic reaction is detected in the presence of a substrate for the enzyme.

11. The method according to any one of claims 1 to 10, wherein
the specimen is a food-derived specimen.

12. The method according to any one of claims 1 to 11, wherein
the target is a peanut allergen, preferably conarachin or a subunit thereof, more preferably Ara h1.

13. The method according to claim 12, wherein
the peanut allergen is a native allergen or a thermally denatured allergen.

14. Use of a target analysis kit in the method according to any one of claims 1 to 13 comprising:
a labeled binding nucleic acid molecule that binds to a target; and
a carrier on which a blocking nucleic acid molecule that binds to the labeled binding nucleic acid molecule is immobilized.

## Patentansprüche

1. Targetanalyseverfahren, umfassend:
Zurreaktionbringen einer Probe eines markierten Nukleinsäurebindemoleküls, das an ein Target bindet, und eines Trägers, auf dem ein Nukleinsäureblockierungsmolekül, das an das markierte Nukleinsäurebindemolekül bindet, immobilisiert ist;
Auftrennen in eine Fraktion aus dem Träger und eine Fraktion aus anderen Bestandteilen als dem Träger; und
Nachweisen einer Markierung des markierten Nukleinsäurebindemoleküls in mindestens einer der Fraktionen aus der Fraktion aus dem Träger oder der Fraktion aus anderen Bestandteilen als dem Träger, um ein Target in der Probe zu analysieren;
wobei
das markierte Nukleinsäurebindemolekül das folgende Polynukleotid (a) umfasst:
(a) das folgende Polynukleotid (a1) oder (a2):
(a1) ein Polynukleotid, bestehend aus einer Basensequenz der SEQ ID NO: 1, und
(a2) ein Polynukleotid, das an ein Erdnussallergen bindet und aus einer Basensequenz besteht, die mindestens 90 % Identität zu der Basensequenz des Polynukleotids (a1) aufweist.

2. Verfahren nach Anspruch 1, umfassend:
Zurreaktionbringen der Probe mit dem markierten Nukleinsäurebindemolekül; und
Zurreaktionbringen einer Mischung aus der Probe und dem markierten Nukleinsäurebindemolekül mit dem Träger.

3. Verfahren nach Anspruch 1 oder 2, wobei
das Nukleinsäureblockierungsmolekül ein Nukleinsäuremolekül ist, das eine Basensequenz umfasst, die zu dem markierten Nukleinsäurebindemolekül komplementär ist.

4. Verfahren nach einem der Ansprüche 1 bis 3, wobei
das Nukleinsäureblockierungsmolekül ein Nukleinsäuremolekül ist, das eine Basensequenz enthält, die zu einer Basensequenz komplementär ist, die an ein Target im markierten Nukleinsäurebindemolekül bindet.

5. Verfahren nach einem der Ansprüche 1 bis 4, wobei
das Nukleinsäureblockierungsmolekül ein Nukleinsäuremolekül ist, das ein Polynukleotid umfasst, das aus einer Basensequenz besteht, die eine Komplementarität in einem Bereich von 5 % bis 100 % bezüglich einer Basensequenz des markierten Nukleinsäurebindemoleküls aufweist.

6. Verfahren nach einem der Ansprüche 1 bis 5, wobei
das Nukleinsäureblockierungsmolekül das folgende Polynukleotid (b) umfasst:
(b) das folgende Polynukleotid (b1) oder (b2):
(b1) ein Polynukleotid, bestehend aus einer Basensequenz der SEQ ID NO: 3, und
(b2) ein Polynukleotid, bestehend aus einer Basensequenz, die mindestens 90 % Identität zu der Basensequenz des Polynukleotids (b1) aufweist.

7. Verfahren nach einem der Ansprüche 1 bis 6, wobei der Träger ein Bead, vorzugsweise ein Polystyrolbead oder ein magnetisches Bead, ist.

8. Verfahren nach Anspruch 7, wobei
die Fraktion aus dem magnetischen Bead und die Fraktion aus anderen Bestandteilen als dem magnetischen Bead voneinander getrennt werden, indem der magnetische Bead unter Verwendung eines magnetischen Körpers abgetrennt wird.

9. Verfahren nach einem der Ansprüche 1 bis 8, wobei
die Markierung ein Enzym, vorzugsweise Luciferase, ist, und
eine enzymatische Reaktion des markierten Nukleinsäurebindemoleküls in mindestens einer der Fraktionen aus der Fraktion aus dem Träger oder der Fraktion aus anderen Bestandteilen als dem Träger nachgewiesen wird.

10. Verfahren nach Anspruch 9, wobei
die enzymatische Reaktion in Gegenwart eines Substrats des Enzyms nachgewiesen wird.

11. Verfahren nach einem der Ansprüche 1 bis 10, wobei
die Probe eine aus Lebensmitteln stammende Probe ist.

12. Verfahren nach einem der Ansprüche 1 bis 11, wobei
das Target ein Erdnussallergen, vorzugsweise Conarachin oder eine Untereinheit davon, bevorzugter Ara h1, ist.

13. Verfahren nach Anspruch 12, wobei
das Erdnussallergen ein natives Allergen oder ein thermisch denaturiertes Allergen ist.

14. Verwendung eines Targetanalysekits in dem Verfahren nach einem der Ansprüche 1 bis 13, umfassend:
ein markiertes Nukleinsäurebindemolekül, das an ein Target bindet; und
einen Träger, auf dem ein Nukleinsäureblockierungsmolekül immobilisiert ist, das an das markierte Nukleinsäurebindemolekül bindet.

## Revendications

1. Procédé d'analyse de cible comprenant :
le fait d'amener un spécimen, une molécule d'acide nucléique de liaison marquée qui se lie à une cible, et un support sur lequel est immobilisée une molécule d'acide nucléique de blocage qui se lie à la molécule d'acide nucléique de liaison marquée à réagir;
la séparation d'une fraction du support et d'une fraction de composants autres que le support l'une de l'autre ; et
la détection d'un marqueur de la molécule d'acide nucléique de liaison marquée dans au moins l'une de la fraction du support ou de la fraction des composants autres que le support pour analyser une cible dans le spécimen,
dans lequel
la molécule d'acide nucléique de liaison marquée comporte le polynucléotide (a) suivant :
(a) le polynucléotide (a1) ou (a2) suivant :
(a1) un polynucléotide consistant en une séquence de base de SEQ ID N° 1, et
(a2) un polynucléotide qui se lie à un allergène d'arachide et consiste en une séquence de base ayant au moins 90% d'identité avec la séquence de base du polynucléotide (a1).

2. Procédé selon la revendication 1, comprenant :
le fait d'amener le spécimen à réagir avec la molécule d'acide nucléique de liaison marquée ; et
le fait d'amener un mélange du spécimen et de la molécule d'acide nucléique de liaison marquée à réagir avec le support.

3. Procédé selon la revendication 1 ou 2, dans lequel
la molécule d'acide nucléique de blocage est une molécule d'acide nucléique qui comporte une séquence de base complémentaire de la molécule d'acide nucléique de liaison marquée.

4. Procédé selon l'une quelconque des revendications 1 à 3, dans lequel
la molécule d'acide nucléique de blocage est une molécule d'acide nucléique qui comporte une séquence de base complémentaire d'une séquence de base qui se lie à une cible dans la molécule d'acide nucléique de liaison marquée.

5. Procédé selon l'une quelconque des revendications 1 à 4, dans lequel
la molécule d'acide nucléique de blocage est une molécule d'acide nucléique qui comporte un polynucléotide consistant en une séquence de base ayant une complémentarité dans une plage de 5 % à 100 % vis-à-vis d'une séquence de base de la molécule d'acide nucléique de liaison marquée.

6. Procédé selon l'une quelconque des revendications 1 à 5, dans lequel
la molécule d'acide nucléique de blocage comporte le polynucléotide (b) suivant :
(b) le polynucléotide (b1) ou (b2) suivant :
(b1) un polynucléotide consistant en une séquence de base de SEQ ID N° 3, et
(b2) un polynucléotide consistant en une séquence de base ayant au moins 90 % d'identité avec la séquence de base du polynucléotide (b1).

7. Procédé selon l'une quelconque des revendications 1 à 6, dans lequel
le support est une bille, de préférence une bille de polystyrène ou une bille magnétique.

8. Procédé selon la revendication 7, dans lequel
la fraction de la bille magnétique et la fraction de composants autres que la bille magnétique sont séparées l'une de l'autre par séparation de la bille magnétique à l'aide d'un corps magnétique.

9. Procédé selon l'une quelconque des revendications 1 à 8, dans lequel
le marqueur est une enzyme, de préférence la luciférase, et
une réaction enzymatique de la molécule d'acide nucléique de liaison marquée dans au moins l'une de la fraction du support ou de la fraction de composants autres que le support est détectée.

10. Procédé selon la revendication 9, dans lequel
la réaction enzymatique est détectée en présence d'un substrat pour l'enzyme.

11. Procédé selon l'une quelconque des revendications 1 à 10, dans lequel
le spécimen est un spécimen d'origine alimentaire.

12. Procédé selon l'une quelconque des revendications 1 à 11, dans lequel
la cible est un allergène d'arachide, de préférence la conarachine ou une sous-unité de celle-ci, de manière encore plus préférée Ara h1.

13. Procédé selon la revendication 12, dans lequel
l'allergène d'arachide est un allergène natif ou un allergène thermiquement dénaturé.

14. Utilisation d'un kit d'analyse de cible dans le procédé selon l'une quelconque des revendications 1 à 13 comprenant :
une molécule d'acide nucléique de liaison marquée qui se lie à une cible ; et
un support sur lequel est immobilisée une molécule d'acide nucléique de blocage qui se lie à la molécule d'acide nucléique de liaison marquée.


### SEQUENCE LISTING

<110> NEC Solution Innovators, Ltd.
<120> Method of analyzing target and kit of analyzing target for use in the same method
<130> 1010100048
<150> JP2016-011024
   <151> 2016-01-22
<160> 6
<170> PatentIn version 3.5
<210> 1
   <211> 79
   <212> DNA
   <213> Artificial Sequence
<220>
   <223> aptamer
<400> 1
   ggatattgcc tcgccacagt taagtcaggt ggttggttat ggttgggact gactctctac 60
   agggaacgct cggattatc 79
<210> 2
   <211> 76
   <212> DNA
   <213> Artificial Sequence
<220>
   <223> aptamer
<400> 2
   ggtaaggtcc tcagtcctcg attagctatc ctcccgtttc ctctactttc tgcgtgatca 60
   cggcggctct cattac 76
<210> 3
   <211> 40
   <212> DNA
   <213> Artificial Sequence
<220>
   <223> complementary polynucleotide
<400> 3
   aaaaaaaaaa aaaaaaaaaa tagagagtca gtcccaacca 40
<210> 4
   <211> 40
   <212> DNA
   <213> Artificial Sequence
<220>
   <223> complementary polynucleotide
<400> 4
   aaaaaaaaaa aaaaaaaaaa agtagaggaa acgggaggat 40
<210> 5
   <211> 19
   <212> DNA
   <213> Artificial Sequence
<220>
   <223> aptamer
<400> 5
   agttaagtca ggtggttgg 19
<210> 6
   <211> 19
   <212> DNA
   <213> Artificial Sequence
<220>
   <223> aptamer
<400> 6
   atcctcccgt ttcctctac 19